(19) **Europäisches Patentamt**

**European Patent Office**

**Office européen des brevets**

(11) **EP 1 270 528 A2**

(12) **EUROPÄISCHE PATENTANMELDUNG**

(43) Veröffentlichungstag:
02.01.2003 Patentblatt 2003/01

(51) Int Cl.⁷: **C04B 24/04**, C07C 67/26, C07C 69/54

(21) Anmeldenummer: 02013434.2

(22) Anmeldetag: **13.06.2002**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **18.06.2001 DE 10129287**

(71) Anmelder: **BASF Aktiengesellschaft**
**67063 Ludwigshafen (DE)**

(72) Erfinder:
• **Zirnstein, Michael, Dr.**
**69198 Schriesheim (DE)**

• **Völkel, Ludwig**
**67117 Limburgerhof (DE)**
• **Kroner, Matthias, Dr.**
**67304 Eisenberg (DE)**
• **Bohres, Edward, Dr.**
**67061 Ludwigshafen (DE)**
• **Oftring, Alfred, Dr.**
**67098 Bad Dürkheim (DE)**

(74) Vertreter: **Isenbruck, Günter, Dr. et al**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

(54) **Verfahren zur Herstellung von Alkylpolyalkylenglycolcarbonsäureestern**

(57) Die vorliegende Erfindung betrifft ein Verfahren zur Herstellung von Alkylpolyalkylenglycolcarbonsäureestern umfassend die Umsetzung eines Carbonsäureesters mit einem Alkylenoxid, wobei für die Umsetzung eine Multimetallcyanid-Verbindung der allgemeinen Formel I

$$M^3_z M^1_a [M^2(CN)_b(A)_c]_d \cdot fM^1_g X_n \cdot mM^3_p Y_q \cdot h(H_2O) \cdot eL \cdot kP \qquad (1)$$

als Katalysator eingesetzt wird, sowie die Verwendung der erfindungsgemäß hergestellten Alkylpolyalkylenglycolcarbonsäureestern als Rohstoffe für Betonverflüssigerpolymere.

Printed by Jouve, 75001 PARIS (FR)

**Beschreibung**

Die vorliegende Erfindung betrifft ein Verfahren zur Herstellung von Alkylpolyalkylenglycolcarbonsäureestern aus Carbonsäureestern und Alkylenoxid, wobei für die Umsetzung eine Multimetallcyanid-Verbindung als Katalysator eingesetzt wird, sowie die Verwendung der erfindungsgemäß hergestellten Alkylpolyalkylenglycolcarbonsäureester als Rohstoffe für Betonverflüssigerpolymere.

Verfahren zur Herstellung von Alkylpolyalkylenglycolcarbonsäureestern unter Verwendung von Katalysatoren sind an sich bekannt. Die Art der verwendeten Katalysatoren variiert dabei stark.

In der EP-A 0 140 545 wird ein Verfahren zur Herstellung von Glycolderivaten aus Epoxiden und Carbonsäureestern unter Verwendung von Amidin-Katalysatoren beschrieben.

Vielfach werden modifizierte Hydrotalcite als Katolysatoren eingesetzt. Die Verwendung calcinierter Hydrotalcite als Katalysator zur Herstellung entsprechender Alkoxylierungsprodukte ist beispielsweise aus der DE-A 19 843 384 oder der DE-A 3 914 131 bekannt. Die DE-A 19 611 999 betrifft ein Verfahren zur Herstellung alkoxylierter Fettsäurealkylester unter Verwendung von mit Lithiumhydroxiden, Erdalkalisalzen oder Zinnsalzen modifizierten Hydrotalciten als Katalysator.

In der DE-A 197 34 906 wird ebenfalls ein Verfahren zur Alkoxylierung von Estern unter Verwendung von modifizierten Hydrotalciten als Katalysator beschrieben. Bei den dort beschriebenen Katalysatoren handelt es sich um aus Polykationen aufgebaute Mischhydroxide.

Darüber hinaus werden als Katalysatoren auch basische Mischkatalysatoren auf Basis von Natrium- und Kaliumhydroxiden, -oxiden, -carbonaten, -alkoholaten oder -carboxylaten verwendet. Die JP 10 099 693 betrifft beispielsweise ein Verfahren zur Alkoxylierung unter Verwendung von Mischkatalysatoren auf Basis von basischen Alkali- oder Erdalkaliverbindungen und ausgewählten Metalloxiden.

Die bekannten Verfahren sind mit Nachteilen behaftet. Insbesondere sind ein hoher Restgehalt des als Edukt eingesetzten Esters und unerwünschte Nebenreaktionen, beispielsweise die Polymerisation von ungesättigten Carbonsäureestern auf Grund der benötigten hohen Oxalkylierungstemperaturen, zu nennen.

Metallcyanid-Verbindungen sind aus dem Stand der Technik als Katalysatoren für Polyadditionen, insbesondere für Ring-öffnende Polymerisationen von Alkylenoxiden bekannt, wie beispielsweise in der EP-A 0 892 002, EP-A 0 862 977 und in der EP-A 0 755 716 beschrieben.

In der WO 99/10407 wird ein Verfahren zur Herstellung von Polyethern mit Hydroxyfunktionalität und ungesättigten Gruppen beschrieben. Die Synthese erfolgt durch Oxyalkylierung eines ungesättigten Monomers, das reaktive Wasserstoffatome aufweist. Die Umsetzung erfolgt in Gegenwart eines Doppelmetallcyanid-Katalysators, dessen Herstellung in der US 5,545,601 näher beschrieben wird.

Ausgehend vom Stand der Technik war es eine Aufgabe der vorliegenden Erfindung, geeignete Katalysatoren für die Umsetzung von Carbonsäureestern mit Alkylenoxiden bereitzustellen, mit denen die gewünschten Alkylpolyalkylenglycolcarbonsäureestern unter milderen Reaktionsbedingungen und unter Vermeidung unerwünschter Nebenprodukte hergestellt werden können.

Erfindungsgemäß wird diese Aufgabe durch ein Verfahren unter Verwendung einer Multimetallcyanid-Verbindung der allgemeinen Formel I als Katalysator gelöst.

Demgemäß betrifft die Erfindung ein Verfahren zur Herstellung von Alkylpolyalkylenglycolcarbonsäureestern umfassend die Umsetzung mindestens eines Carbonsäureesters mit mindestens einem Alkylenoxid, wobei für die Umsetzung eine Multimetallcyanid-Verbindung der allgemeinen Formel I als Katalysator eingesetzt wird:

$$M^3_z M^1_a [M^2(CN)_b(A)_c]_d \cdot f M^1_g X_n \cdot m M^3_p Y_q \cdot h(H_2O) \cdot eL \cdot kP \qquad (I),$$

in der

- M$^1$ mindestens ein Metallion, ausgewählt aus der Gruppe bestehend aus $Zn^{2+}$, $Fe^{2+}$, $Fe^{3+}$, $Co^{3+}$, $Ni^{2+}$, $Mn^{2+}$, $Co^{2+}$, $Sn^{2+}$, $Pb^{2+}$, $Mo^{4+}$, $Mo^{6+}$, $Al^{3+}$, $V^{4+}$, $V^{5+}$, $Sr^{2+}$, $W^{4+}$, $W^{6+}$, $Cr^{2+}$, $Cr^{3+}$, $Cd^{2+}$, $Hg^{2+}$, $Pd^{2+}$, $Pt^{2+}$, $V^{2+}$, $Mg^{2+}$, $Ca^{2+}$, $Ba^{2+}$, $Cu^{2+}$ ist,

- M$^2$ mindestens ein Metallion, ausgewählt aus der Gruppe bestehend aus $Fe^{2+}$, $Fe^{3+}$, $Co^{2+}$, $Co^{3+}$, $Mn^{2+}$, $Mn^{3+}$, $V^{4+}$, $V^{5+}$, $Cr^{2+}$, $Cr^{3+}$, $Rh^{3+}$, $Ru^{2+}$, $Ir^{3+}$ ist,

- M$^1$ und M$^2$ gleich oder verschieden sind,

- M$^3$ mindestens ein Metallion, ausgewählt aus der Gruppe bestehend aus Li$^+$, Na$^+$, K$^+$, Rb$^+$, Cs$^+$, Mg$^{2+}$, Ca$^{2+}$, Ba$^{2+}$, Sr$^{2+}$, Ammonium-Ionen der allgemeinen Formel R$^1$R$^2$R$^3$R$^4$N$^+$ ist, wobei R$^1$, R$^2$, R$^3$ und R$^4$ H oder ein Kohlenwasserstoffrest mit 1 bis 6 C-Atomen sind,

- A, X und Y unabhängig voneinander ein Anion, ausgewählt aus der Gruppe, bestehend aus Halogenid, Hydroxid, Alkoholat, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,

- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,

- k eine gebrochene oder ganze Zahl größer oder gleich Null ist, und

- P ein organischer Zusatzstoff ist,

- a, b, c, d, g, n, p, q und z so ausgewählt sind, daß die Elektroneutralität der Verbindung (I) gewährleistet ist, wobei c oder z oder c und z 0 sein können,

- e die Anzahl der Ligandenmoleküle eine gebrochenen oder ganze Zahl größer 0 oder 0 ist,

- f, k, h und m unabhängig voneinander eine gebrochene oder ganze Zahl größer 0 oder 0 sind.

Als organische Zusatzstoffe P sind zu nennen: Polyether, Polyester, Polycarbonate, Polyalkylenglykolsorbitanester, Polyakylenglykolglycidylether, Polyacrylamid, Poly(acrylamid-co-acrylsäure), Polyacrylsäure, Poly(acrylamid-comaleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose, Polyacetate, ionische oberflächen- und grenzflächenaktive Verbindungen, Gallensäure oder deren Salze, Ester oder Amide, Carbonsäureester mehrwertiger Alkohole und Glycoside.

Mit dem erfindungsgemäßen Verfahren gelingt es, Alkylpolyalkylenglycolcarbonsäureester formal im Sinne einer Insertionsreaktion durch Oxalkylierung ausgehend von Carbonsäureestern und Alkylenoxiden mit einem erfindungsgemäßen Katalysatorsystem in hoher Ausbeute und mit vollständigem Umsatz bezüglich des Carbonsäureesters herzustellen.

Im Rahmen der vorliegenden Erfindung können als Carbonsäureester solche ohne funktionelle Gruppen mit aktivem Wasserstoff, beispielsweise ohne HydroxyGruppen, eingesetzt werden. Es ist jedoch im Rahmen der vorliegenden Erfindung ebenso möglich, daß Carbonsäureester mit funktionellen Gruppen mit aktivem Wasserstoff eingesetzt werden, sofern es nicht zu unerwünschten Nebenreaktionen kommt. Darüber hinaus können im Rahmen der vorliegenden Erfindung auch Carbonsäureester mit verschiedenen Funktionellen Gruppen mit aktivem Wasserstoff oder ohne aktiven Wasserstoff eingesetzt werden.

In einer bevorzugten Ausführungsform betrifft die Erfindung ein Verfahren zur Herstellung von Alkylpolyalkylenglycolcarbonsäureestern, wobei eine oder mehrere der folgenden Eigenschaften erfüllt sind:

(A) M$^1$ ist ausgewählt aus der Gruppe Zn$^{2+}$, Fe$^{2+}$, Fe$^{3+}$, Co$^{3+}$, Ni$^{2+}$, Mn$^{2+}$, Co$^{2+}$;

(B) M$^2$ ist ausgewählt aus der Gruppe Fe$^{2+}$, Fe$^{3+}$, Co$^{3+}$;

(C) M$^3$ ist ausgewählt aus der Gruppe Na$^+$, K$^+$, Cs$^+$, Ammonium-Ion der allgemeinen Formel R$^1$R$^2$R$^3$R$^4$N$^+$.

Im Rahmen der vorliegenden Erfindung ist es weiter bevorzugt, daß mindestens M$^1$ oder M$^2$ Fe$^{2+}$ oder Fe$^{3+}$ ist, insbesondere zusammen mit den unter (A) bis (C) genannten weiteren bevorzugten Metallionen.

In einer weiteren bevorzugten Ausführungsform der vorliegenden Erfindung sind sowohl M$^1$ als auch M$^2$ Fe$^{2+}$ oder Fe$^{3+}$, insbesondere zusammen mit den unter (C) genannten weiteren bevorzugten Metallionen für M$^3$.

Geeignet sind im Rahmen der vorliegenden Erfindung beispielsweise Verbindungen der allgemeinen Formel II:

$$M^a Fe[Fe(CN)_6] \, nH_2O \qquad \text{(II)},$$

wobei $M^a$ Kalium ($K^+$) oder Ammonium ($NH_4^+$)sein kann, wobei im Rahmen der vorliegenden Erfindung $K^+$ gegenüber $NH_4^+$ bevorzugt ist.

Erfindungsgemäß können als Katalysator auch Verbindungen der Formel III:

$$Fe_4[Fe(CN)_6]_3 \qquad \text{(III)}$$

eingesetzt werden, wobei auch weitere Ionen wie bezüglich der Formel (I) definiert, im Austausch von einem oder bis zu drei Fe(III)-Ionen enthalten sein können.

Als im Rahmen der vorliegenden Erfindung als Katalysator besonders geeignet haben sich beispielsweise die folgenden Eisenhexacyanoferrate erwiesen: Eisenblau Pigmente, Eisencyanblau, Vossen-Blau®, Preussisch Blau, Berliner Blau, Turnbullsblau, Milori Blau, Pariser Blau. Ebenfalls geeignet sind beispielsweise Panax Blue®, Manox Blau® oder Sicomet® Blau.

Durch die erfindungsgemäße Verwendung der Multimetallcyanid-Verbindungen können die gewünschten Alkylpolyalkylenglycolcarbonsäureester unter milden Reaktionsbedingungen durch eine Oxalkylierungsreaktion hergestellt werden.

Die Multimetallcyanid-Verbindungen werden in der Regel durch Reaktion mindestens eines Metallsalzes mit mindestens einer Cyanometall-Verbindung erzeugt. Als Cyanometall-Verbindung können beispielsweise Salze oder Säuren verwendet werden. Bei dem erfindungsgemäßen Verfahren stören Verunreinigungen durch Alkali- oder Erdalkali-Metallsalze nicht, so daß eine aufwendige und kostspielige Reinigung der Katalysatoren entfällt.

In einer bevorzugten Ausführungsform betrifft die Erfindung ein Verfahren zur Herstellung von Alkylpolyalkylenglycolcarbonsäureestern, wobei die als Katalysator eingesetzte Multimetallcyanid-Verbindung kristallin, teilkristallin, amorph oder teilweise amorph ist.

Es ist dabei im Rahmen der Erfindung möglich, daß zunächst eine Katalysator-Vorläuferverbindung hergestellt wird, die dann beispielsweise durch Oxidation, Reduktion, Umkristallisation oder andere Reaktionen in die eigentlich katalytisch aktive Verbindung überführt wird. Somit ist es im Rahmen der vorliegenden Erfindung beispielsweise auch denkbar, daß die Vorläuferverbindung in die Reaktion eingesetzt wird und die eigentlich katalytisch aktive Verbindung erst im Reaktionsmedium in Gegenwart der umzusetzenden Komponenten erzeugt wird.

Ferner besteht die Möglichkeit, durch Zusatz geeigneter Stoffe, wie beispielsweise oberflächenaktiver Substanzen, die Morphologie der Multimetallcyanid-Teilchen so zu steuern, daß eine erhöhte Aktivität für die zu katalysierende Reaktion erzielt wird.

Im Rahmen der vorliegenden Erfindung werden Katalysator-Mengen von 0,001 bis 30 Gew.-%, bevorzugt von 0,01 bis 10 Gew.-%, insbesondere bevorzugt 0,1 bis 5 Gew.-% oder 0,2 bis 3 Gew.-%, jeweils bezogen auf die Menge des eingesetzten Carbonsäureesters, verwendet.

Insbesondere betrifft die Erfindung daher ein Verfahren zur Herstellung von Alkylpolyalkylenglycolcarbonsäureestern, wobei der Katalysator in Mengen von 0,01 bis 30 Gew.-%, bezogen auf die Menge des eingesetzten Carbonsäureesters, eingesetzt wird.

Prinzipiell können für das erfindungsgemäße Verfahren Ester aller substituierten und unsubstituierten verzweigten oder unverzweigten Carbonsäuren eingesetzt werden, sofern die funktionellen Gruppen des Carbonsäureesters nicht die katalysierte Reaktion beeinträchtigen.

Die Alkylkomponente des Carbonsäureesters ist im Rahmen der vorliegenden Erfindung insbesondere ein verzweigter oder unverzweigter gesättigter Alkylrest mit 1 bis 22 C-Atomen oder ein verzweigter oder unverzweigter, ein- oder mehrfach ungesättigter Alkylrest mit 2 bis 22 C-Atomen, besonders bevorzugt ein Methylrest.

Bevorzugt werden im Rahmen der vorliegenden Erfindung Ester der folgenden Carbonsäuren eingesetzt: substituierte oder unsubstituierte, gesättigte oder ungesättigte Monocarbonsäuren mit 3 bis 22 C-Atomen, substituierte oder unsubstituierte, gesättigte Dicarbonsäuren mit 2 bis 36 C-Atomen, substituierte oder unsubstituierte, ungesättigte Dicarbonsäuren mit 4 bis 36 C-Atomen und substituierte oder unsubstituierte aromatische Mono- und Dicarbonsäuren.

In einer weiteren Ausführungsform betrifft die Erfindung demgemäß ein Verfahren zur Herstellung von Alkylpolyalkylenglycolcarbonsäureestern, wobei sich der Carbonsäureester ableiten läßt von Carbonsäuren ausgewählt aus der Gruppe bestehend aus substituierten oder unsubstituierten, gesättigten oder ungesättigten Monocarbonsäuren mit 3 bis 22 C-Atomen, substituierte oder unsubstituierte, gesättigte Dicarbonsäuren mit 2 bis 36 C-Atomen, substituierte oder unsubstituierte, ungesättigte Dicarbonsäuren mit 4 bis 36 C-Atomen und substituierten oder unsubstituierten aromatischen Mono- und Dicarbonsäuren.

Insbesondere sind als erfindungsgemäß besonders bevorzugte Carbonsäureester solche zu nennen, die sich von den folgenden Carbonsäuren ableiten: ungesättigte substituierte oder unsubstituierte Monocarbonsäuren mit 3 bis 5 C-Atomen und ungesättigte substituierte oder unsubstituierte Dicarbonsäuren mit 4 bis 8 C-Atomen, beispielsweise Acrylsäure, Methacrylsäure oder Crotonsäure, Fumarsäure, Maleinsäure oder Itaconsäure; gesättigte substituierte oder unsubstituierte Monocarbonsäuren mit 1 bis 5 C-Atomen und gesättigte substituierte oder unsubstituierte Dicarbonsäuren mit 2 bis 5 C-Atomen, beispielsweise Ameisensäure, Essigsäure, Propionsäure, Pivalinsäure, Oxalsäure, Malonsäure oder Bernsteinsäure.

Weiterhin bevorzugt sind außerdem gesättigte oder ungesättigte substituierte oder unsubstituierte Monocarbonsäuren mit 6 bis 22 C-Atomen, die auch cycloaliphatische Strukturelemente aufweisen können, beispielsweise Hexansäure, Heptansäure, Cyclohexancarbonsäure, 2-Ethylhexansäure, Caprinsäure (C10), Myristinsäure (C14), Palmitinsäure (C16), Stearinsäure (C18), Ölsäure, Behensäure (C22); gesättigte oder ungesättigte substituierte oder unsubstituierte Dicarbonsäuren mit 6 bis 36 C-Atomen, die insbesondere cycloaliphatische Strukturelemente aufweisen, beispielsweise Adipinsäure, Pimelinsäure (C7), Azelainsäure (C9), Sebacinsäure (C10), Dimerfettsäuren mit 36 C-Atomen; substituierte oder unsubstituierte aromatische Mono- und Dicarbonsäuren, beispielsweise Benzoesäure, Phthalsäure, Isophthalsäure, Terephthalsäure oder Naphthalincarbonsäuren.

Ganz besonders bevorzugt ist die Verwendung von Acrylsäure- und Methacrylsäureestern. Daher betrifft die Erfindung in einer bevorzugten Ausführungsform ein Verfahren zur Herstellung von Alkylpolyalkylenglycolcarbonsäureestern, wobei der Carbonsäureester Acrylsäuremethylester oder Methacrylsäuremethylester ist.

Für das erfindungsgemäße Verfahren können prinzipiell alle Alkylenoxide eingesetzt werden, die dem Fachmann bekannt sind. Beispielsweise werden substituierte oder unsubstituierte Alkylenoxide mit 2 bis 24 C-Atomen, bevorzugt Alkylenoxide mit Halogen-, Hydroxy-, nicht cyclische Ether- oder Ammoniumsubstituenten. Insbesondere sind zu nennen: aliphatische 1,2-Alkylenoxide mit 2 bis 4 C-Atomen, beispielsweise Ethylenoxid, Propylenoxid, 1,2-Butylenoxid, 2,3-Butylenoxid oder Isobutylenoxid, aliphatisch 1,2-Alkylenoxide mit 5 bis 24 C-Atomen, cycloaliphatisch Alkylenoxide, beispielsweise Cyclopentenoxid, Cyclohexenoxid oder Cyclododecatrien-(1,5,9)-monoxid, araliphatische Alkylenoxide, beispielsweise Styroloxid.

Bevorzugte substituierte Alkylenoxide sind beispielsweise Epichlorhydrin, Epibromhydrin, 2,3-Epoxy-1-propanol, 1-Allyloxy-2,3-epoxypropan, 2,3-Epoxypropylphenylether, 2,3-Epoxypropyl-isopropylether, 2,3-Epoxypropyloctylether oder 2,3-Epoxypropyltrimethyl-ammoniumchlorid.

Besonders bevorzugt werden für das erfindungsgemäße Verfahren 1,2-Alkylenoxide mit 2 bis 4 C-Atomen eingesetzt, insbesondere Ethylenoxid oder Propylenoxid.

In einer bevorzugten Ausführungsform betrifft die Erfindung daher ein Verfahren zur Herstellung von Alkylpolyalkylenglycolcarbonsäureestern aus einem Carbonsäureester und einem Alkylenoxid, wobei das Alkylenoxid ein 1,2-Alkylenoxid mit 2 bis 4 C-Atomen ist.

Insbesondere bevorzugt wird als Alkylenoxid Ethylenoxid oder Propylenoxid eingesetzt.

Im Rahmen der vorliegenden Erfindung werden der Carbonsäureester und das Alkylenoxid in einem Verhältnis von 1:1 bis 1:200, bevorzugt in einem Verhältnis von 1:5 bis 1:100 und besonders bevorzugt in einem Verhältnis von 1:10 bis 1:50 eingesetzt.

Die Umsetzung des Carbonsäureesters mit dem Alkylenoxid kann im Rahmen der vorliegenden Erfindung bei 20 bis 200°C durchgerührt werden. Bevorzugt ist ein Temperaturbereich von 40 bis 150°C, insbesondere von 50 bis 120°C. Die Umsetzung kann sowohl bei Atmosphärendruck oder bei Unterdruck, als auch bei erhöhtem Druck, beispielsweise bei einem Druck von 0,8 bis 50 bar, insbesondere bei einem Druck von 1 bis 10 bar durchgeführt werden.

Daher betrifft die Erfindung auch ein Verfahren zur Herstellung von Alkylpolyalkylenglycolcarbonsäureestern, wobei die Temperatur bei der Umsetzung des Carbonsäureesters mit dem Alkylenoxid 50 bis 120°C beträgt.

In einer weiteren Ausführungsform betrifft die Erfindung ein Verfahren zur Herstellung von Alkylpolyalkylenglycolcarbonsäureestern, wobei bei der Umsetzung des Carbonsäureesteres mit dem Alkylenoxid ein Druck von 1 bis 10 bar herrscht.

Die Umsetzung kann bei dem erfindungsgemäßen Verfahren im Sinne eines Batch-Verfahrens oder kontinuierlich durchgeführt werden. Sie kann in einem Rührreaktor, Rohrreaktor, Schlaufenreaktor, in einem Festbettreaktor oder in einem Fließbettreaktor durchgeführt werden.

Bei der Umsetzung des Carbonsäureesters mit einem Alkylenoxid können dem Fachmann bekannte Hilfs- und Zusatzstoffe zugesetzt werden. In einer bevorzugten Ausführungsform wird die Umsetzung des Carbonsäureesters mit dem Alkylenoxid in Gegenwart mindestens eines Polymerisationsinhibitors durchgeführt. Als Polymerisationsinhibitoren können beispielsweise Hydrochinon, Hydrochinonmonomethylether, 2,5-Di-t-butylhydrochinon, 2,6-Di-t-butyl-p-cresol, Nitrosoverbindungen wie Isoacrylnitrit, Nitrosodiphenylamin oder N-Nitrosocyclohexylhydroxylamin, Methylenblau, Phenothiazin, Gerbsäure oder Diphenylamin eingesetzt werden. Es ist im Rahmen der vorliegenden Erfindung auch möglich, daß zwei oder mehr dieser Polymerisationsinhibitoren eingesetzt werden. Die Polymerisationsinhibitoren werden in Mengen von 10 bis 50000 ppm, insbesondere von 100 bis 10000 ppm, jeweils bezogen auf den eingesetzten Carbonsäureester, eingesetzt.

Ferner können im Rahmen der vorliegenden Erfindung zusätzlich geringe, sicherheitstechnisch unbedenkliche Anteile molekularer Sauerstoff oder Stickstoffmonoxid verwendet werden.

Es ist für das erfindungsgemäße Verfahren nicht notwendig, Lösungsmittel für die Umsetzung des Carbonsäureesters mit dem Alkylenoxid zu verwenden. Es ist aber ebenso möglich, das erfindungsgemäße Verfahren in Gegenwart von Wasser oder organischen Lösungsmitteln wie beispielsweise aliphatischen, cycloaliphatischen oder aromatischen Kohlenwasserstoffen, Alkoholen, Ethern, Acetalen, Ketonen, Estern oder cyclischen Carbonaten durchzuführen.

Die Abtrennung des Katalysators vom Reaktionsprodukt kann erfindungsgemäß beispielsweise durch Filtration erfolgen, insbesondere Tiefenfiltration, Querstromfiltration, Membranfiltration oder Ultrafiltration.

Die erfindungsgemäß hergestellten Alkylpolyalkylenglycolcarbonsäureester können beispielsweise als Monomere für radikalische Homo- oder Copolymerisationen eingesetzt werden. Diese Homo- oder Copolymere können beispielsweise als Betonverflüssigerpolymere eingesetzt werden. Daher betrifft die vorliegende Erfindung auch die Verwendung der erfindungsgemäß hergestellten Alkylpolyalkylenglycolcarbonsäureester als Rohstoffe für Betonverflüssigerpolymere.

Nachfolgend wird die Erfindung anhand von Beispielen näher erläutert.

BEISPIELE

Beispiel 1 (Katalysatorherstellung)

Zu 211 g einer 30%-igen wäßrigen Lösung von Kaliumhexacyanoferrat(II)-Trihydrat wurden unter Rühren 180 g einer 30%-igen wäßrigen Lösung von Eisen(III)chlorid-Hexahydrat zugetropft. Eine halbe Stunde wurde nachgerührt und anschließend über eine Filternutsche abgesaugt. Der Filterrückstand wurde zweimal mit Methanol gewaschen, indem eine entsprechende Aufschlämmung jeweils 30 Minuten gerührt und dann abgesaugt wurde. Bei 50°C im Vakuum wurde getrocknet. Es wurden 77,4 g eines dunkelblauen Pulvers erhalten.

Die Elementaranalyse des erhaltenen Produkts ergab folgende Zusammensetzung: C: 18,5%, H: 1,4%, N: 20,0%, O: 9,4%, Fe: 30,0%, K: 12,2%, Cl: 8,2%.

Beispiel 2 (Oxalkylierungsreaktion)

In 120 g Methacrylsäuremethylester wurden 4,8 g Phenothiazin gelöst und 4,8 g des gemäß Beispiel 1 hergestellten Katalysators suspendiert. Diese Mischung wurde in einem Druckreaktor bei 80°C mit 866 g Ethylenoxid begast, wobei der Druck auf maximal 8,0 atm anstieg. Diese Mischung wurde 18 Stunden gerührt, wobei die Reaktormanteltemperatur im Bereich von 75 bis 81 °C gehalten wurde und der Druck auf 3,8 atm abfiel. Es wurden 922 g Austrag erhalten, aus denen Reste von Ethylenoxid im Vakuum entfernt wurden. Das Produkt erstarrte beim Abkühlen zu einem wachsartigen Feststoff.

Das Produkt wies eine sehr enge Molmassenverteilung auf, wobei im Mittel 14 Ethylenoxid-Moleküle in die Esterbindung insertiert wurden. Es konnte kein Methacrylsäureester nachgewiesen werden, d.h. der Umsatz bezüglich des Methacrylsäureesters war vollständig.

**Patentansprüche**

1. Verfahren zur Herstellung von Alkylpolyalkylenglycolcarbonsäureestern umfassend die Umsetzung mindestens eines Carbonsäureesters mit mindestens einem Alkylenoxid, wobei für die Umsetzung eine Multimetallcyanid-Verbindung der allgemeinen Formel I als Katalysator eingesetzt wird:

$$M^3_z M^1_a [M^2(CN)_b(A)_c]_d \cdot fM^1_g X_n \cdot mM^3_p Y_q \cdot h(H_2O) \cdot eL \cdot kP \qquad (I),$$

in der

- $M^1$ mindestens ein Metallion, ausgewählt aus der Gruppe bestehend aus $Zn^{2+}$, $Fe^{2+}$, $Fe^{3+}$, $Co^{3+}$, $Ni^{2+}$, $Mn^{2+}$, $Co^{2+}$, $Sn^{2+}$, $Pb^{2+}$, $Mo^{4+}$, $Mo^{6+}$, $Al^{3+}$, $V^{4+}$, $V^{5+}$, $Sr^{2+}$, $W^{4+}$, $W^{6+}$, $Cr^{2+}$, $Cr^{3+}$, $Cd^{2+}$, $Hg^{2+}$, $Pd^{2+}$, $Pt^{2+}$, $V^{2+}$, $Mg^{2+}$, $Ca^{2+}$, $Ba^{2+}$, $Cu^{2+}$ ist,

- $M^2$ mindestens ein Metallion, ausgewählt aus der Gruppe bestehend aus $Fe^{2+}$, $Fe^{3+}$, $Co^{2+}$, $Co^{3+}$, $Mn^{2+}$, $Mn^{3+}$, $V^{4+}$, $V^{5+}$, $Cr^{2+}$, $Cr^{3+}$, $Rh^{3+}$, $Ru^{2+}$, $Ir^{3+}$ ist,

- M$^1$ und M$^2$ gleich oder verschieden sind,

- M$^3$ mindestens ein Metallion, ausgewählt aus der Gruppe bestehend aus Li$^+$, Na$^+$, K$^+$, Rb$^+$, Cs$^+$, Mg$^{2+}$, Ca$^{2+}$, Ba$^{2+}$, Sr$^{2+}$, Ammonium-Ionen der allgemeinen Formel R$^1$R$^2$R$^3$R$^4$N$^+$ ist, wobei R$^1$, R$^2$, R$^3$ und R$^4$ H oder ein Kohlenwasserstoffrest mit 1 bis 6 C-Atomen sind,

- A, X und Y unabhängig voneinander ein Anion, ausgewählt aus der Gruppe, bestehend aus Halogenid, Hydroxid, Alkoholat, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,

- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,

- k eine gebrochene oder ganze Zahl größer oder gleich Null ist, und

- P ein organischer Zusatzstoff ist,

- a, b, c, d, g, n, p, q und z so ausgewählt sind, daß die Elektroneutralität der Verbindung (I) gewährleistet ist, wobei c oder z oder c und z 0 sein können,

- e die Anzahl der Ligandenmoleküle eine gebrochenen oder ganze Zahl größer 0 oder 0 ist,

- f, k, h und m unabhängig voneinander eine gebrochene oder ganze Zahl größer 0 oder 0 sind.

2. Verfahren zur Herstellung von Alkylpolyalkylenglycolcarbonsäureestern nach Anspruch 1, wobei eine oder mehrere der folgenden Eigenschaften erfüllt sind:

    (A) M$^1$ ist ausgewählt aus der Gruppe Zn$^{2+}$, Fe$^{2+}$, Fe$^{3+}$, Co$^{3+}$, Ni$^{2+}$, Mn$^{2+}$, Co$^{2+}$;

    (B) M$^2$ ist ausgewählt aus der Gruppe Fe$^{2+}$, Fe$^{3+}$, Co$^{3+}$;

    (C) M$^3$ ist ausgewählt aus der Gruppe Na$^+$, K$^+$, Ammonium-Ion der allgemeinen Formel R$^1$R$^2$R$^3$R$^4$N$^+$.

3. Verfahren zur Herstellung von Alkylpolyalkylenglycolcarbonsäureestern nach einem der Ansprüche 1 oder 2, wobei M$^1$ oder M$^2$ Fe$^{2+}$ oder Fe$^{3+}$ist.

4. Verfahren zur Herstellung von Alkylpolyalkylenglycolcarbonsäureestern nach einem der vorherigen Ansprüche, wobei sowohl M$^1$ als auch M$^2$ Fe$^{2+}$ oder Fe$^{3+}$ ist.

5. Verfahren zur Herstellung von Alkylpolyalkylenglycolcarbonsäureestern nach einem der vorherigen Ansprüche, wobei die Multimetallcyanid-Verbindung kristallin ist.

6. Verfahren zur Herstellung von Alkylpolyalkylenglycolcarbonsäureestern nach einem der vorherigen Ansprüche, wobei der Katalysator in Mengen von 0,01 bis 30 Gew.-% bezogen auf die Menge des Carbonsäureesters eingesetzt wird.

7. Verfahren zur Herstellung von Alkylpolyalkylenglycolcarbonsäureestern nach einem der vorherigen Ansprüche, wobei der Carbonsäureester Acrylsäuremethylester oder Methacrylsäuremethylester ist.

8. Verfahren zur Herstellung von Alkylpolyalkylenglycolcarbonsäureestern nach einem der vorherigen Ansprüche, wobei das Alkylenoxid Ethylenoxid oder Propylenoxid ist.

9. Verfahren zur Herstellung von Alkylpolyalkylenglycolcarbonsäureestern nach einem der vorherigen Ansprüche, wobei die Temperatur bei der Umsetzung 50 bis 120°C beträgt oder bei der Umsetzung des Carbonsäureesters mit dem Alkylenoxid ein Druck von 1 bis 10 bar herrscht oder beides.

10. Verwendung eines Alkylpolyalkylenglycolcarbonsäureestern, hergestellt nach einem Verfahren gemäß einem der Ansprüche 1 bis 9, als Rohstoff für Betonverflüssigerpolymere.